# FASCICULE DE BREVET EUROPEEN

(11) **EP 3 030 909 B1**
(45) Date de publication et mention de la délivrance du brevet: **20.02.2019**
(21) Numéro de dépôt: 14749836.4
(22) Date de dépôt: 08.08.2014
(51) Int. Cl.: G01N 35/10, G01N 15/06, G01N 9/00

(54) **PROCÉDÉ DE PRÉPARATION D'UN CONTENANT CELLULAIRE COMPRENANT DES MOYENS DE PRÉ-ANALYSE D'UN ÉCHANTILLON PRÉLEVÉ**
VERFAHREN ZUR HERSTELLUNG EINES ZELLBEHÄLTERS MIT MITTEL ZUR VORANALYSE EINER ENTNOMMENEN PROBE
METHOD FOR PREPARING A CELLULAR CONTAINER COMPRISING MEANS FOR PRE-ANALYSIS OF A SAMPLE THAT HAS BEEN TAKEN

(30) Priorité: 09.08.2013 FR 1357924
(43) Date de publication de la demande: 15.06.2016
(73) Titulaire: Novacyt, 78140 Velizy Villacoublay (FR)
(72) Inventeur: PELTIER, Eric, F-92140 Clamart (FR)
(74) Mandataire: Lavoix
(86) Numéro de dépôt international: PCT/EP2014/067116
(87) Numéro de publication internationale: WO 2015/018933

(56) Documents cités:
- EP-A1- 2 535 712
- EP-A2- 0 556 971
- WO-A1-2011/117523
- US-A1- 2007 041 875
- US-A1- 2007 084 990
- US-A1- 2010 163 111
- US-A1- 2010 221 772
- US-A1- 2010 288 941
- US-A1- 2012 142 043

## Description

La présente invention concerne un procédé de préparation à l'analyse d'une suspension cellulaire tel que défini dans les revendications.

Le procédé est par exemple destinés à préparer une lame d'analyse cellulaire dans le cadre d'un dépistage ou d'un diagnostic médical à partir de prélèvements cytologiques, tels que des frottis du col de l'utérus ou autres.

Les cellules prélevées sont disposées dans un flacon de prélèvement où les cellules sont mises en solution. Une partie de la solution cellulaire est ensuite prélevée et disposée dans un contenant d'analyse en vue d'une analyse diagnostique du prélèvement. Le document WO-2011/117523 décrit par exemple un tel appareil de préparation.

Cependant, le contenant d'analyse obtenu ne permet pas toujours d'obtenir un diagnostic pertinent et sûr, par exemple parce que l'échantillon prélevé ne contient pas suffisamment de cellules ou parce que les cellules intéressantes en vue d'un diagnostic ne se trouvent pas dans cet échantillon. Le contenant d'analyse obtenu est alors inutilisable.

Dans certaines applications comme le frottis de dépistage du cancer du col de l'utérus, des étalements cellulaires sont réalisés sur des lames d'analyse et chaque étalement doit contenir un nombre de cellules satisfaisant la classification de Bethesda, qui est une classification pour standardiser le résultat diagnostic des frottis. Chaque étalement doit ainsi comprendre plus de 5000 cellules.

Dans le document FR-2 922 019, est divulgué un procédé de mesure de la densité cellulaire qui se réalise dans la chambre de décantation située au-dessus de la lame d'analyse. La densité mesurée, par exemple par diffraction de lumière, est par la suite comparée par rapport à une densité seuil correspondant à la densité cellulaire minimale de l'étalement sur la lame d'analyse nécessaire pour réaliser un diagnostic pertinent. Ainsi, si la densité mesurée est inférieure à la densité seuil, le procédé comprend une étape additionnelle d'ajustement de la densité cellulaire de l'étalement par l'ajout dans la chambre de décantation, d'une quantité plus importante de suspension cellulaire prélevée dans le flacon que la quantité standard afin de réaliser une deuxième lame d'étalement représentative.

Toutefois, ce type de mesure dans la chambre de décantation présente l'inconvénient d'être réalisé après un premier dépôt de cellules. Effectivement, en cas d'insuffisance de cellules sur la lame d'analyse, il est nécessaire de prélever à nouveau de la suspension cellulaire pour réaliser un deuxième étalement. Il s'agit donc de réaliser une étape supplémentaire complète de dépôt de cellules sur lame. D'autres exemples de documents de l'état de la technique sont : US2012/0142043, US2010/0163111, EP2535712, US2007/0041875 ou US2010/0221772.

L'un des buts de l'invention est de pallier ces inconvénients en proposant un procédé de préparation permettant d'ajuster la densité cellulaire et de réduire le temps de préparation de contenants d'analyse à une seule étape standardisée, tout en garantissant une qualité de ces contenants permettant l'obtention d'un diagnostic fiable.

A cet effet, l'invention a pour objet le procédé d'analyse ou de contrôle pré-analytique de préparation d'une suspension cellulaire du type précité, dans lequel entre l'étape de prélèvement et l'étape de dépôt, le procédé comprend au moins une étape de pré-analyse de l'échantillon prélevé, ladite étape étant réalisée par un dispositif de pré-analyse disposé sur la tubulure du dispositif de pipetage-distribution.

Ce procédé permet de garantir la qualité des contenants en donnant l'information pré-analytique de la suspension cellulaire du prélèvement fait par le praticien, c'est-à-dire dans le flacon. En analysant l'échantillon prélevé, par exemple pour déterminer s'il contient suffisamment de cellules, avant de réaliser le contenant d'analyse, on garantit que l'échantillon utilisé pour réaliser ce contenant est bien pertinent. Cette pré-analyse est réalisée dans le dispositif de pipetage-distribution ce qui évite de réaliser un deuxième étalement d'analyse si la pré-analyse n'est pas satisfaisante. On évite la réalisation d'une seconde lame par le dispositif de pipetage-distribution et on garantit la réalisation de contenants d'analyse aptes à être analysés ultérieurement de façon fiable dans une étape pré-analytique et non post-analytique.

Suivant d'autres aspects de l'invention, le procédé comporte l'une ou plusieurs des caractéristiques suivantes, prise(s) isolément ou suivant toutes les combinaisons techniquement possibles :
- l'étape de mesure de la densité cellulaire comprend en outre une étape de comparaison de la densité cellulaire mesurée à une densité de référence ;
- si la densité cellulaire mesurée est inférieure, respectivement supérieure, à la densité de référence, l'étape (a) comprend en outre au moins les étapes suivantes :
   (f) prélèvement d'un volume de la suspension cellulaire supérieur à un volume standard, respectivement inférieur au volume standard, au moyen du dispositif de pipetage-distribution de sorte à obtenir des étalements de densité homogène ;
   (g) établissement d'une information relative au volume prélevé afin d'assurer une traçabilité des étapes de traitement de l'échantillon,
   lesdites étapes étant réalisées jusqu'à ce qu'un seuil du nombre de cellules prélevées garantissant la pertinence de l'analyse soit atteint ;
- la densité cellulaire est mesurée en émettant de la lumière dans la tubulure au travers de l'échantillon et en mesurant l'atténuation ou la diffraction de la lumière ayant traversée l'échantillon ; et
- le dispositif de pipetage-distribution comprend au moins une aiguille adaptée pour prélever un échantillon de la suspension, la tubulure s'étendant entre l'aiguille et des moyens d'aspiration-refoulement, l'étape (a) de prélèvement étant agencée pour qu'au moins une partie de l'échantillon prélevé passe dans la tubulure en regard du dispositif de pré-analyse.

L'invention sera mieux comprise à la lecture de la description détaillée qui va suivre, donnée uniquement à titre d'exemple, faite en référence aux dessins annexés sur lesquels :
- la figure 1 est une vue en coupe schématique d'un automate de prélèvement et d'analyse comprenant un appareil de préparation à l'analyse et
- la figure 2 est une vue en coupe d'un flacon au cours d'une étape de prélèvement du procédé de préparation à l'analyse d'une suspension cellulaire selon l'invention.

Dans la description, les termes « amont » et « aval » sont définis par rapport à la direction de circulation d'un liquide détergent dans l'aiguille, le liquide étant refoulé dans l'aiguille par des moyens de refoulement disposés en amont de l'aiguille. Par conséquent, la dispense de l'aiguille se fait selon une direction amont-aval, tandis que l'aspiration se fait selon une direction aval-amont.

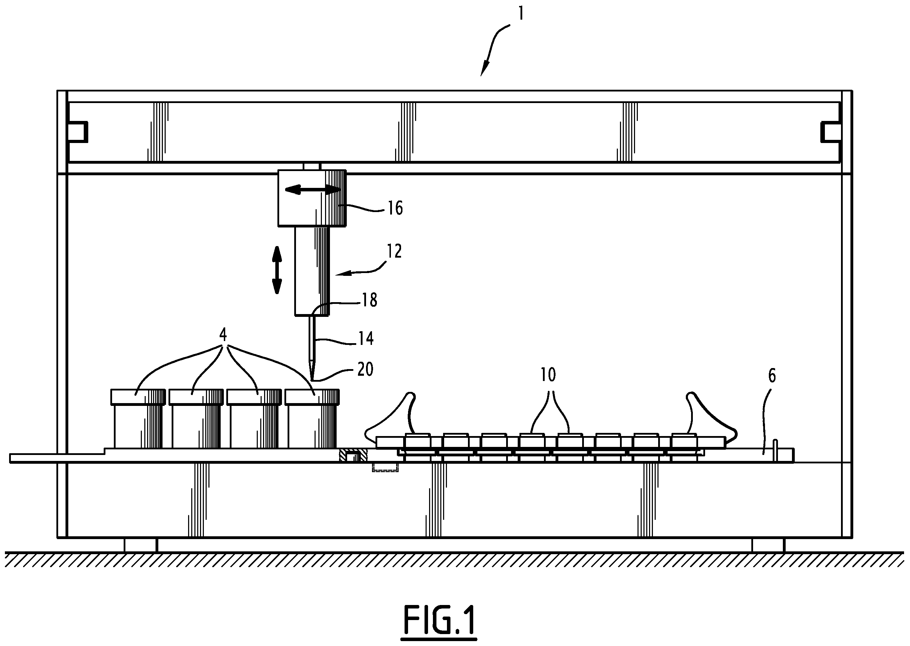

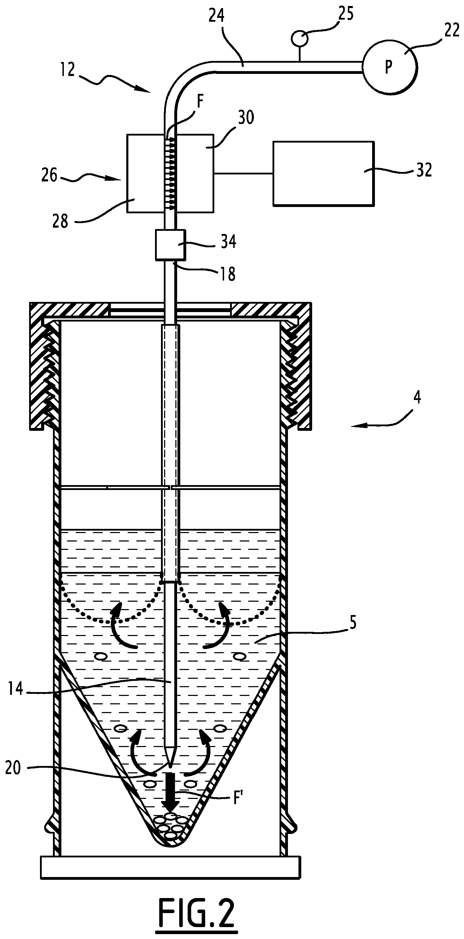

Sur la Fig. 1, on a représenté un automate 1 de préparation et d'analyse d'au moins un flacon 4 contenant une suspension cellulaire 5 à analyser (Fig. 2), telle que par exemple une suspension cytologique fixée. Les suspensions cellulaires 5 sont obtenues par une mise en suspension de prélèvements cellulaires obtenus par exemple au cours d'une opération de dépistage par frottis du col de l'utérus ou par d'autres types de prélèvements. La façon dont le prélèvement est mis en suspension et dont les cellules sont préservées est connue et ne sera pas décrite en détail ici.

L'automate 1 comprend pour l'essentiel un plateau de réception 6 des flacons 4 et de chambres de décantation 10 disposées au-dessus de lames d'analyse sur lesquelles des étalements cellulaires en couche mince doivent être réalisés à partir des suspensions cellulaires 5, par l'intermédiaire d'un dispositif de pipetage-distribution 12, apte à prélever des échantillons des suspensions cellulaires 5 et à déposer ces échantillons dans les chambres de décantation 10, en passant éventuellement par des étapes de traitement intermédiaires.

Dans la mesure où l'automate 1 et son fonctionnement ont été décrits dans le document WO-2011/117523, l'automate ne sera pas décrit plus en détail ici. L'homme du métier peut se référer à ce document pour en connaître toutes les autres caractéristiques, détails et variantes de réalisation. Il est bien entendu que l'automate peut être adapté pour réaliser d'autres types de contenants d'analyse que des lames d'analyse. Par exemple, les contenants d'analyse 22 de ce système 20 de préparation et d'analyse peuvent comporter, selon le mode d'analyse choisi par le praticien, des lames d'étalement, des tubes de prélèvement ou d'aliquotage et des puits d'analyse ou de décantation.

Le dispositif de pipetage-distribution 12, ou encore dispositif de prélèvement, s'étend au-dessus du plateau de réception 6 et est apte à prélever, déplacer et à verser ou évacuer diverses produits liquides, notamment des échantillons des suspensions cellulaires 5. Pour ce faire, le dispositif de pipetage-distribution 12 comprend une pluralité d'aiguilles 14, ou pipettes, tubulaires et creuses, déplaçables entre les différents postes de l'automate 1 par un bras 16 et mobile verticalement pour permettre d'abaisser et de relever les aiguilles 14.

Chaque aiguille 14 s'étend entre une extrémité amont 18 et une extrémité aval 20 en communication fluidique l'une avec l'autre.

Les opérations de prélèvement d'échantillons d'une suspension cellulaire 5 se font par aspiration par l'extrémité aval 20 de l'aiguille 14 et les opérations de versement ou d'évacuation se font par refoulement, ou dispense, par cette extrémité aval 20. Pour ce faire, l'extrémité amont 18 de l'aiguille est en communication fluidique avec des moyens d'aspiration et de refoulement 22 disposés en amont de l'aiguille 14 et agencés pour permettre une aspiration de produits et le refoulement de ces produits par l'extrémité aval 20 de l'aiguille. Pour ce faire, les moyens d'aspiration et de refoulement 22 sont reliés à l'extrémité amont 18 de l'aiguille 14 par une tubulure 24, par exemple une tubulure souple permettant le déplacement de l'aiguille 14 par rapport aux moyens d'aspiration et de refoulement 22, comme représenté sur la Fig. 2.

Les moyens d'aspiration et de refoulement 22 sont par exemple formés par une pompe d'aspiration-refoulement classique pour ce genre d'application. Alternativement, les moyens d'aspiration et de refoulement 22 pourraient être formés par un système de piston et/ou de valves, ou autre. Les moyens d'aspiration et de refoulement 22 permettent de mettre en oeuvre les étapes impliquant le dispositif de pipetage-distribution 12 dans le fonctionnement de l'automate 1. Un capteur de niveau 25 est prévu au niveau de la tubulure 24 entre l'extrémité amont 18 de l'aiguille 14 et les moyens d'aspiration et de refoulement 22 afin de déterminer la présence ou non de produits dans la tubulure 24.

Le dispositif de pipetage-distribution 12 comprend également des moyens de pré-analyse 26 de l'échantillon prélevé par l'aiguille 14. Ces moyens de pré-analyse 26 sont prévus sur la tubulure 24, c'est-à-dire en amont de l'aiguille 14 et en aval des moyens d'aspiration et de refoulement 22, par exemple au voisinage de l'extrémité amont 18 de l'aiguille 14.

Ces moyens de pré-analyse sont adaptés pour permettre une pré-analyse d'un échantillon prélevé immédiatement de la suspension cellulaire 5, comme représenté sur la Fig. 2, ou immédiatement après une étape de traitement de l'échantillon, telle qu'une étape de marquage cellulaire. Par marquage cellulaire, on entend l'introduction d'un marqueur particulier dans l'échantillon ou dans la suspension cellulaire ou une étape de coloration de l'échantillon ou de la suspension cellulaire. Cette pré-analyse consiste en une mesure de la densité cellulaire de l'échantillon.

Les moyens de pré-analyse 26 comprennent au moins des moyens d'émission 28 d'un signal F, des moyens de réception 30 du signal F ayant traversé la tubulure 24 et des moyens d'analyse 32 du signal reçu par les moyens de réception 30. Les moyens d'émission 28 et les moyens de réception 30 sont agencés de part et d'autre de la tubulure 24 de sorte qu'un signal émis par les moyens d'émission 28 soit reçu de l'autre côté de la tubulure 24 par les moyens de réception 30 après que le signal F a traversé la tubulure 24 et son contenu.

A titre d'exemple, les moyens d'émission 28 et de réception 30 pourraient être prévus dans un connecteur 34 agencé pour fixer ensemble l'aiguille 14 et la tubulure 24 ou être prévus dans un manchon destiné à entourer la tubulure 24.

Pour la mesure d'une densité cellulaire, les moyens d'émission 28 sont par exemple une source de lumière, telle qu'une ou plusieurs diodes électroluminescentes, laser (laser accordable, source de lumière polychromatique, etc), agencée pour émettre un signal lumineux au travers de la tubulure et les moyens de réception 30 sont par exemple un capteur du signal lumineux, tel qu'une photodiode (APD, barrette CCD, caméra CCD CMOS ou spectromètre), ayant traversé la tubulure.

Les moyens d'analyse 32 sont alors par exemple des moyens de mesure de l'absorption du signal lumineux reçu par les moyens de réception 30 ou des moyens de mesure de la diffraction de la lumière par les cellules présentes dans l'échantillon pré-analysé. De telles méthodes de mesure de la densité cellulaire, par mesure de l'absorption de la lumière ou de la diffraction, sont connues et ne seront pas décrites plus en détail ici.

L'étape de pré-analyse est réalisée de la façon suivante : l'échantillon prélevé est aspiré dans la tubulure au moins jusqu'à ce qu'il passe en regard des moyens de pré-analyse 26, ce qui peut être déterminé grâce au capteur de niveau 24. Les moyens de pré-analyse sont alors déclenchés et le signal reçu, modifié par rapport au signal émis suite à son passage au travers de l'échantillon, est transmis aux moyens d'analyse 32, qui détermine la densité cellulaire de l'échantillon.

Dans le cas de la mesure de la densité cellulaire dans l'échantillon, les moyens d'analyse 32 compare la densité mesurée à une densité dite de référence. La densité de référence est la densité cellulaire nécessaire à avoir pour obtenir une densité cellulaire et un nombre de cellules pertinents dans le contenant d'analyse 22 pour pouvoir réaliser un diagnostic.

Si, dans un premier cas, la densité cellulaire mesurée est supérieure à la densité de référence, l'aiguille 14 prélève du flacon un volume de la suspension cellulaire inférieur au volume standard, normalement prélevé lorsque la densité cellulaire correspond à la densité de référence. Puis, l'aiguille 14 se déplace, via le bras robotisé 16, au-dessus d'un contenant d'analyse 10 ou d'autres parties de l'automate si des étapes de traitement de l'échantillon sont prévues. L'échantillon contenu dans la tubulure 24 est versé dans le contenant d'analyse 10 ou est traité, par exemple dans un puits ou tube de réception pour technique ou analyse complémentaire ou autre.

Si dans un deuxième cas, la densité cellulaire mesurée est inférieure à la densité de référence, l'aiguille 14 prélève du flacon un volume de la suspension cellulaire supérieur au volume standard de sorte à augmenter le nombre de cellules prélevées et arriver à un seuil garantissant la pertinence de l'analyse.

L'appareil et le procédé de préparation à l'analyse de suspension cellulaire décrits ci-dessus, permettent d'obtenir directement dans les contenants d'analyse des échantillons cellulaires optimisés pour réaliser une analyse pertinente menant à une interprétation du dépistage ou du diagnostic. L'échantillon est analysé immédiatement avant son prélèvement ou avant son traitement, ce qui évite ainsi la réalisation d'un second étalement.

La détermination de la densité cellulaire permet une caractérisation pré-analytique de la suspension cellulaire et l'établissement d'une information sur le volume de la suspension prélevé afin d'assurer une traçabilité des étapes de traitement de l'échantillon afin d'informer la personne ou les moyens en charge de l'analyse sur la représentativité du prélèvement initial.

Les moyens de pré-analyse 26 peuvent également être utilisés pour assurer une vérification du bon fonctionnement de l'automate 1. En effet, si l'automate est agencé pour réalisé un traitement de la suspension cellulaire, par exemple une coloration ou un marquage, il est possible de déterminer si ce traitement a été correctement réalisé en faisant passer un échantillon issu de la suspension traitée par les moyens de pré-analyse 26 et ainsi vérifier que les caractéristiques de l'échantillon obtenu à l'issu du traitement correspondent bien aux caractéristiques attendues suite à l'opération de traitement. Les moyens de pré-analyse permettent donc un suivi qualitatif de l'automate.

De plus, l'appareil peut être adapté à une pluralité d'aiguilles, de sorte de pouvoir réaliser simultanément plusieurs pré-analyses, et donc en particulier, d'automatiser la préparation et l'analyse d'une pluralité de suspensions.

## Revendications

1. Procédé de préparation à l'analyse d'une suspension cellulaire (5) comprenant au moins les étapes successives suivantes :
(a) prélèvement d'un échantillon de la suspension cellulaire (5) au moyen d'un dispositif de pipetage-distribution (12), ledit dispositif de pipetage-distribution (12) comprenant au moins une tubulure (24) ;
(b) dépôt de l'échantillon présent dans le dispositif de pipetage-distribution (12) dans un contenant d'analyse (10) ;
ledit procédé entre l'étape de prélèvement et l'étape de dépôt, comprend au moins une étape de pré-analyse de l'échantillon prélevé, ladite étape étant réalisée par un dispositif de pré-analyse (26) disposé sur la tubulure (24) du dispositif de pipetage-distribution (12), **caractérisé en ce que** ladite étape de pré-analyse est une étape de mesure de la densité cellulaire de l'échantillon prélevé.

2. Procédé selon la revendication 1, **caractérisé en ce que** l'étape de mesure de la densité cellulaire comprend en outre une étape de comparaison de la densité cellulaire mesurée à une densité de référence.

3. Procédé selon la revendication 2, **caractérisé en ce que**, si la densité cellulaire mesurée est inférieure, respectivement supérieure, à la densité de référence, l'étape (a) comprend en outre au moins les étapes suivantes :
(f) prélèvement d'un volume de la suspension cellulaire supérieur à un volume standard, respectivement inférieur au volume standard, au moyen du dispositif de pipetage-distribution (12) de sorte à obtenir des étalements de densité homogène.
(g) établissement d'une information relative au volume prélevé afin d'assurer une traçabilité des étapes de traitement de l'échantillon,
lesdites étapes étant réalisées jusqu'à ce qu'un seuil du nombre de cellules prélevées garantissant la pertinence de l'analyse soit atteint.

4. Procédé selon l'une quelconque des revendications 1 à 3, **caractérisé en ce que** la densité cellulaire est mesurée en émettant de la lumière dans la tubulure au travers de l'échantillon et en mesurant l'atténuation ou la diffraction de la lumière ayant traversée l'échantillon.

5. Procédé selon l'une quelconque des revendications 1 à 4, **caractérisé en ce que** le dispositif de pipetage-distribution (12) comprend au moins une aiguille (14) adaptée pour prélever un échantillon de la suspension, la tubulure (24) s'étendant entre l'aiguille (14) et des moyens d'aspiration-refoulement (22), l'étape (a) de prélèvement étant agencée pour qu'au moins une partie de l'échantillon prélevé passe dans la tubulure (24) en regard du dispositif de pré-analyse (26).

## Patentansprüche

1. Verfahren zur Herstellung einer Zellsuspension (5) für die Analyse, mindestens die folgenden sukzessiven Schritte umfassend:
(a) Entnehmen einer Probe der Zellsuspension (5) mittels einer Pipettier-Dispensionsvorrichtung (12), wobei die Pipettier- Dispensionsvorrichtung (12) mindestens eine Rohrleitung (24) umfasst;
(b) Ablegen der in der Pipettier- Dispensionsvorrichtung (12) vorhandenen Probe in einem Analysebehältnis (10);
das Verfahren zwischen dem Schritt des Entnehmens und dem Schritt des Ablegens mindestens einen Schritt der Voranalyse der entnommenen Probe umfasst, wobei dieser Schritt durch eine Voranalysevorrichtung (26) vorgenommen wird, der auf der Rohrleitung (24) der Pipettier-Dispensionsvorrichtung (12) angeordnet ist, **dadurch gekennzeichnet, dass** der Schritt der Voranalyse ein Schritt der Zelldichtemessung der entnommenen Probe ist.

2. Verfahren nach Anspruch 1, **dadurch gekennzeichnet, dass** der Schritt der Zelldichtemessung außerdem einen Schritt des Vergleichens der gemessenen Zelldichte mit einer Referenzdichte umfasst.

3. Verfahren nach Anspruch 2, **dadurch gekennzeichnet, dass**, wenn die gemessene Zelldichte kleiner beziehungsweise größer als die Referenzdichte ist, der Schritt (a) außerdem mindestens die folgenden Schritte umfasst:
(f) Entnehmen einer Menge der Zellsuspension, das größer als eine Standardmenge beziehungsweise kleiner als eine Standardmenge ist, mittels der Pipettier-Dispensionsvorrichtung (12) derart, dass Verteilungen homogener Dichte erhalten werden,
(g) Festlegen einer Information bezüglich zur entnommenen Menge, um eine Nachverfolgbarkeit der Schritte zum Behandeln der Probe sicherzustellen,
wobei die genannten Schritte durchgeführt werden, bis eine Schwelle der Anzahl entnommener Zellen, die die Signifikanz der Analyse garantiert, erreicht wird.

4. Verfahren nach einem beliebigen der Ansprüche 1 bis 3, **dadurch gekennzeichnet, dass** die Zelldichte derart gemessen wird, dass Licht in der Rohrleitung durch die Probe hindurch emittiert wird und die Schwächung oder Beugung des Lichts, das die Probe durchquert hat, gemessen wird.

5. Verfahren nach einem beliebigen der Ansprüche 1 bis 4, **dadurch gekennzeichnet, dass** die Pipettier-Dispensionsvorrichtung (12) mindestens eine Nadel (14) umfasst, die geeignet ist, eine Probe der Suspension zu entnehmen, wobei die Rohrleitung (24) sich zwischen der Nadel (14) und Mitteln (22) zum Saugen-Pumpen erstreckt, wobei der Schritt (a) zum Entnehmen derart eingerichtet ist, dass mindestens ein Teil der entnommenen Probe in der Rohrleitung (24) an der Voranalysevorrichtung (26) vorbeigeführt wird.

## Claims

1. A method for preparing a cell suspension (5) for analysis, comprising at least the following successive steps:
(a) taking a sample of the cell suspension (5) using a pipetting-dispensing device (12), said pipetting-dispensing device (12) comprising at least one pipe (24);
(b) depositing the sample present in the pipetting-dispensing device (12) in an analysis container (10);
said method between the sample-taking step and the depositing step comprising at least one pre-analysis step of the sample that has been taken, said step being carried out by means of a pre-analysis device (26) placed on the pipe (24) of the pipetting-dispensing device (12), **characterized in that** said pre-analysis step is a step of measuring the cell density of the sample that has been taken.

2. The method according to claim 1, **characterized in that** the step of measuring the cell density further comprises a step of comparing the measured cell density to a reference density.

3. The method according to claim 2, **characterized in that**, if the measured cell density is lower, respectively higher, than the reference density, step (a) further comprises at least one of the following steps:
(f) taking a volume of the cell suspension larger than a standard volume, respectively smaller than the standard volume, using the pipetting-dispensing device (12) so as to obtain smears with a homogenous density.
(g) establishing information relative to the volume that has been taken in order to ensure traceability of the sample handling steps,
said steps being carried out until a threshold of the number of cells that have been taken guaranteeing the relevance of the analysis is reached.

4. The method according to any one of claims 1 to 3, **characterized in that** the cell density is measured by emitting light in the pipe through the sample and measuring the attenuation or diffraction of light having crossed through the sample.

5. The method according to any one of claims 1 to 4, **characterized in that** the pipetting-dispensing device (12) comprises at least one needle (14) suitable for taking a sample of the suspension, the pipe (24) extending between the needle (14) and suction-discharge means (22), the sample-taking step (a) being arranged so that at least part of the sample that has been taken passes through the pipe (24) across from the pre-analysis device (26).
